# EUROPEAN PATENT SPECIFICATION

(11) **EP 1 321 455 B1**
(45) Date of publication and mention of the grant of the patent: **02.02.2005**
(21) Application number: 02026160.8
(22) Date of filing: 25.11.2002
(51) Int. Cl.: C07C 213/08

(54) **A process for the preparation of 1-(3-trifluoromethylphenyl)-2-(2-benzoyloxyethylamino)-propane**
Verfahren zur Herstellung von 1-(3-Trifluormethylphenyl)-2-(2-benzoyloxyethylamino)-propan
Procédé pour la préparation du (3-trifluorométhylphényl)-1-(2-(benzoyloxy)éthylamino)-2-propane

(30) Priority: 11.12.2001 IT MI20012597
(43) Date of publication of application: 25.06.2003
(73) Proprietor: Synteco S.p.A., 27028 S. Martino Siccomario (IT)
(72) Inventor: Maggi, Domenico, 27028 S. Martino Siccomario (PV) (IT); Bombeccari, Ferdinando, 27028 S. Martino Siccomario (PV) (IT)
(74) Representative: Minoja, Fabrizio, Dr.

(56) References cited:
- ES-A- 474 498
- FR-A- 1 517 587
- US-A- 4 237 165
- P. CALDIROLA ET AL.: "New prenylamine-analogues:...." EUR. J. MED. CHEM., vol. 28, 1993, pages 555-568, XP001109656
- SAKURAI S ET AL: "NOVEL PHENOXYALKYLAMINE DERIVATIVES. VII 1) SYNTHESIS AND PHARMACOLOGICAL ACTIVITIES OF 2-ALKOXY-5-(PHENOXYALKYLAMINO)ALKYL BENZENESULFONAMIDE DERIVATIVES" CHEMICAL AND PHARMACEUTICAL BULLETIN, PHARMACEUTICAL SOCIETY OF JAPAN. TOKYO, JP, vol. 40, no. 6, 1 June 1992 (1992-06-01), pages 1443-1451, XP002051236 ISSN: 0009-2363
- MARCH JERRY: "Advanced organic chemistry, third Edition" 1985 , JOHN WILEY & SONS, INC. , NEW YORK .CHICHESTER . TORONTO . SINGAPORE XP002230660 * page 798 - page 799 *

## Description

The present invention relates to a process for the preparation of 1-(3-trifluoromethylphenyl)-2-(-2-benzoyloxyethylamino)propane, particularly of the highly pure crystalline hydrochloride, starting from 2-benzoylethanolamine hydrochloride and m-trifluoromethylphenylacetone.

### TECHNICAL BACKGROUND

1-(3-Trifluoromethyl)-2-(2-benzoyloxyethylamino)propane (**1**), 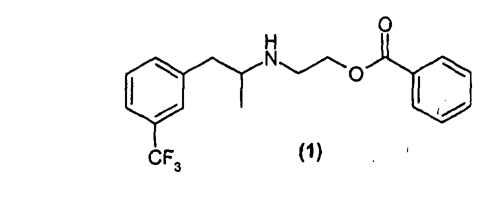 known as Benfluorex, is a hypolipemizing compound first described by Beregi et al. (French Patent 1,517,587 corresponding to US 3,607, 909), whose synthesis comprises reacting 1-(3-trifluoromethylphenyl)-2-amino propane (**2**) with ethylene oxide and acylating the resulting 1-(3-trifluoromethylphenyl)-2(β-hydroxy-ethyl)aminopropane (**3**) with benzoyl chloride, according to scheme 1.

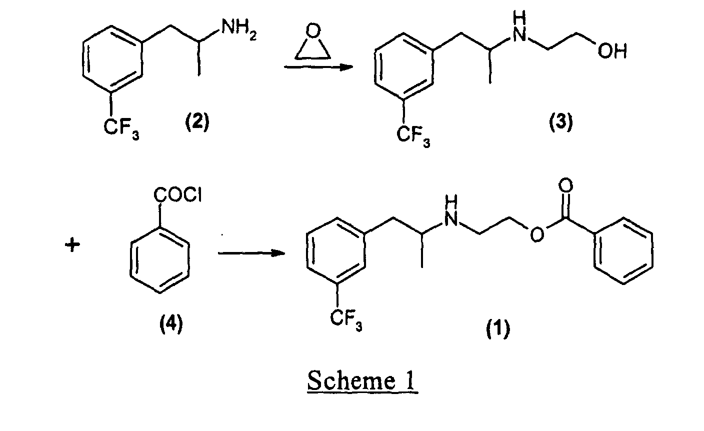

This process has however some drawbacks in that the reaction with ethylene oxide also yields remarkable amounts of bis-hydroxyethyl derivative and the esterification reaction also leads to benzoylation of the amino nitrogen. These by-products have to be removed, thus affecting yields which are unsatisfactory.

Said drawbacks have been overcome by the process described by Dalla Croce (Spanish Patent 474,498), in which the Schiff base (**7**) obtained from m-trifluoromethylphenylacetone (**5**) and benzylamine (**6**) is reduced with NaBH₄, the amino nitrogen of compound (**8**) is alkylated with ethylene oxide, the resulting compound (**9**) is esterified with benzoyl chloride and compound (**10**) is debenzylated by treatment with H₂/Pd (scheme 2).

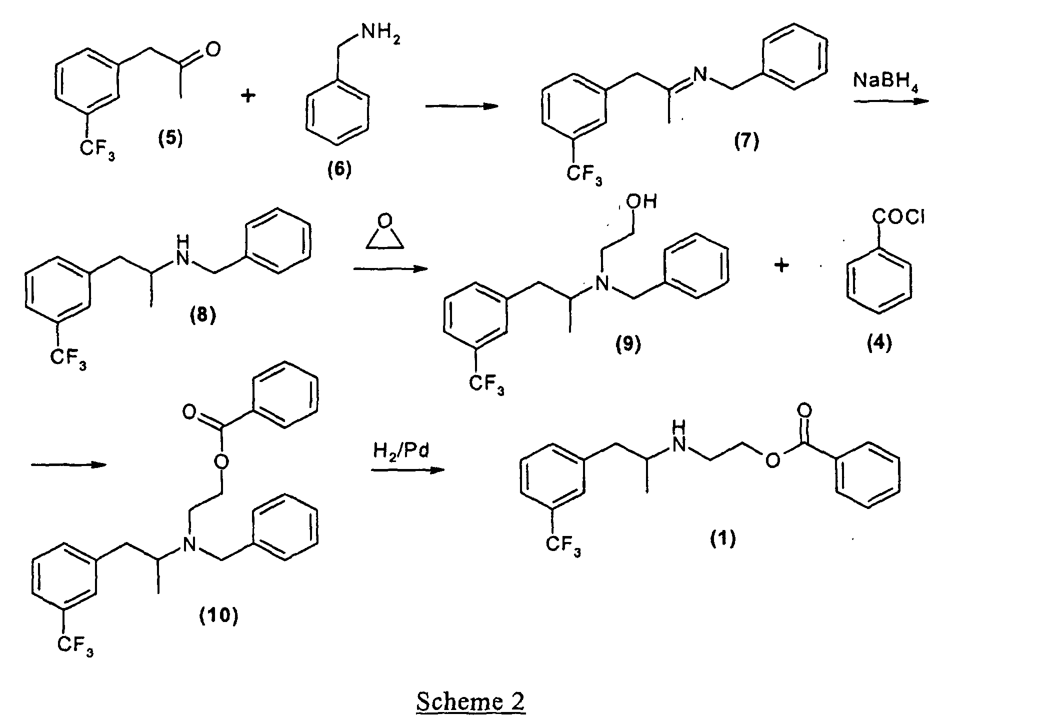

This process, though providing high yields, still involves the risks connected with the use of ethylene oxide, a toxic, flammable gas which is also cancerogenic and mutagenic.

The process of the present invention allows to prepare compound (1) without using said gas and to recover the product as a crystalline hydrochloride having total impurities content below 0.2%.

### DETAILED DISCLOSURE OF THE INVENTION

The present invention relates to a process for the preparation of 1-(3-trifluoromethyl)-2-(2-benzoyloxyethylamino)propane (**1**) which comprises reacting m-trifluoromethylphenylacetone (**5**) with 2-benzoylethanolamine hydrochloride (**11**) in the presence of sodium borohydride in dimethylformamide. The reaction proceeds through formation of ketimine (**12**) which is not isolated from the reaction medium, as illustrated in Scheme 3.

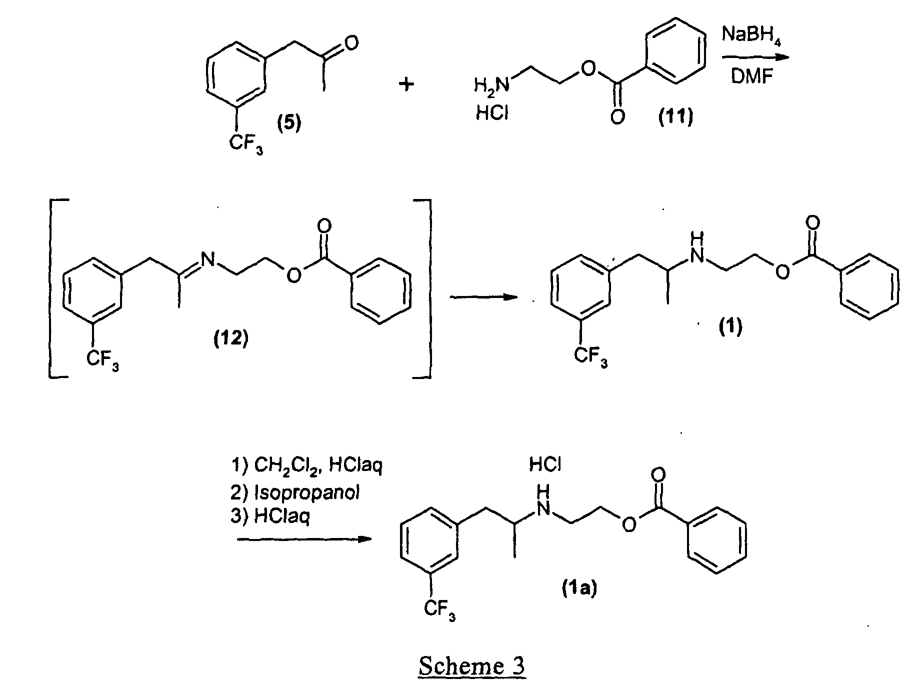

According to a particularly preferred embodiment of the invention, compound (**1**) is recovered as hydrochloride (**1a**) in single crystalline form with high purity. For this purpose, the process comprises the following steps (scheme 3):
a) treatment of crude compound (**1**) with a hydrochloric acid aqueous solution to give hydrochloride (**1a**);
b) purification of hydrochloride (**1a**) from the previous step, by treatment with isopropyl alcohol;
c) crystallization of hydrochloride (**1a**) by treatment with a hydrochloric acid aqueous solution.

2-Benzoylethanolamine hydrochloride (**11**) is prepared without solvent, at temperatures ranging from 120 to 170°C, equimolar amounts of benzoyl chloride and ethanolamine hydrochloride, recovering the product with toluene and washing it while hot with acetone.

The reaction of m-trifluoromethylphenylacetone (**5**) with 2-benzoylethanolamine hydrochloride (**11**) is carried out mixing the reagents in a ratio ranging from 1.75:3.25 to 2.25:2.75, preferably 2:3, in dimethylformamide (DMF), heating the solution to a temperature ranging from 100 to 120°C and slowly adding a solution of NaBH₄ in DMF, under stirring. NaBH₄ will be used in molar ratios ranging from 0.8 to 1:1 with respect to m-trifluoromethylphenylacetone (**5**). The amount of DMF for the preparation of each solution will be 8-12 times the weight of sodium borohydride, and equivalent to 1.1 - 1.3 times the weight of reagents (**5**) and (**1**).

At the end of the addition, stirring is continued until completion of the reaction, namely when HPLC analysis shows a content in compound (**5**) below 1%.

The evaporation residue is dissolved in methylene chloride (CH₂Cl₂) and the resulting solution is treated with a mixture of CH₂Cl₂, demineralised water and 37% hydrochloric acid, in a ratio ranging from 0.5:0.8:0.1 to 0.5:1.2:0.1, preferably 0.5:1:0.1. The organic phase is then separated and the aqueous phase is extracted two further times with CH₂Cl₂; the combined organic phases are washed repeatedly, preferably 3 times, with demineralised water in order to remove the salts and the hydrochloric acid excess.

The evaporation residue is dissolved in isopropyl alcohol, or in a mixture of ethyl acetate and isopropyl alcohol in ratios ranging from 8:1 to 12:1, preferably 10:1. The residue is completely dissolved by heating at temperatures between 40 and 50°C, then the solution is cooled and the precipitate is filtered and washed with the same solvent. The resulting compound (**1a**) consists of two different crystalline forms, as evidenced by X-ray diffractometric analysis (figure 1). D.S.C. analysis (differential scanning calorimetry, figure 2) evidences the softening point of the mass at 154°C and the melting point at 161/163°C. The impurities total content in the product is lower than 0.2%, each single impurity not exceeding 0.1%. Within this limit is, in particular, N-(2-hydroxyethyl)-N-[(1R,S)-1-methyl-2-[3-(trifluoromethyl)phenyl]ethylbenzamide (**13**), named "impurity E" according to the European Pharmacopoeia.

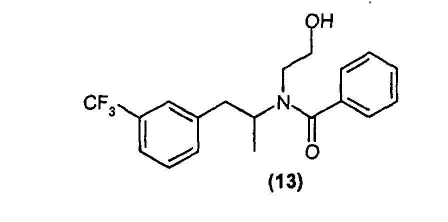

When the impurities total content exceeds 0.2%, which happens rarely, the product can be again washed with isopropyl alcohol or with a mixture of ethyl acetate and isopropyl alcohol in the above cited ratios, preferably in 10:1 ratio.

A single crystalline form can be obtained by hot crystallization of the product directly from the previous step, i.e. not dried, with 10 - 12 volumes of water containing 37% hydrochloric acid in an amount so as to keep pH below 3. Such amount usually ranges from 0.2 to 0.8% by volume with respect to water, and is preferably 0.3%. Crystallographic analysis (figure 3) shows that the product consists of a single crystalline form, which has melting point at 161 - 163°C (figure 4), without previous softening.

The process provides therefore 1-(3-trifluoromethyl)-2-(2-benzoyloxyethylamino)propane hydrochloride with purity above 99.8%, in a single crystalline form, which is also an object of the present invention.

The invention will be further illustrated by the following example.

### EXAMPLE

### Preparation of 1-(3-trifluoromethylphenyl)-2-(-2-benzoyloxyethylamino)propane.

A mixture of 195 kg (967 mol) of benzoylethanolamine hydrochloride and 130 kg (643 mol) of m-trifluoromethylphenylacetone in 360 kg of dimethylformamide is heated to 107 (±5)°C in a reactor. Keeping the temperature at 100 (±5)°C, a solution of 26 kg (690 mol) of sodium borohydride in 290 kg of dimethylformamide is slowly added to the mixture, which is stirred for about 20 min, then cooled to 80 (±5)°C. Completion of the reaction is monitored by HPLC (content in m-trifluoromethylphenylacetone below 1%). HPLC analysis is carried out using an eluent mixture consisting of 50% acetonitrile and 50% buffer phosphate, prepared by dissolving 4.76 g of monobasic potassium phosphate in 1 1 of water and adjusting pH to 3 with 85% phosphoric acid.

The solvent is evaporated off under reduced pressure, atmospheric pressure is restored by bubbling nitrogen and the mass is cooled to 25 (±5)°C. 750 kg of CH₂Cl₂ are added and the mixture is kept under stirring for 5 min, then subjected to the subsequent step.

### Preparation of 1-(3-trifluoromethylphenyl)-2-(-2-benzoyloxyethylamino)propane hydrochloride

550 kg of CH₂Cl₂, 1100 kg of deionized water and 110 kg of 37% hydrochloric acid are loaded in an enameled reactor, then the mixture from the previous step is added, adjusting pH to 1 - 2, if necessary, with 37% hydrochloric acid. The suspension is stirred for 6-10 hours keeping the temperature at 20°C (±5)°C, then the organic phase is separated and the aqueous phase is extracted twice with 250 kg of CH₂Cl₂. The combined organic phases are washed three times with water, each time stirring for 15 (±5) min. the two phases then leaving them to separate for the same time.

### Crystallization of 1-(3-trifluoromethylphenyl)-2-(-2-benzoyloxyethylamino)propane hydrochloride

The organic phase from the previous step is concentrated by heating to 45 (±5)°C; 400 kg of hot isopropyl alcohol are added to complete dissolution, then the mixture is cooled with water-jacket (water temperature first 25 (±5)°C, then 6° (±2)°C) and left under stirring for 1 hour (±10 min.).

The precipitate is centrifuged and washed with isopropyl alcohol.

The product is then placed in a stainless steel reactor of suitable size, containing ten volumes of deionized water acidified with 0.03 volumes of 37% hydrochloric acid. The mixture is then heated to 95 (±5)°C for 15 (±5) min. and isopropyl alcohol is distilled off, then the mixture is cooled to 10 (±5)°C in 3 h (±30 min) while stirring, and left under stirring for a further 30 (±5) min.

The precipitate is then centrifuged, washed with deionized water, filtered and dried at 70 (±10)°C under vacuum for 10 (± 2) h.

Overall yield: 120-130% by weight with respect to m-trifluoromethylphenylacetone.

## Claims

1. A process for the preparation of 1-(3-trifluoromethylphenyl)-2-(2-benzoyloxyethylamino)propane, comprising the reaction between m-trifluoromethylphenylacetone and 2-benzoylethanolamine hydrochloride in the presence of NaBH₄ and in DMF as solvent.

2. A process as claimed in claim 1, further comprising:
a) treatment of crude compound (1) obtained according to the process of claim 1 with a hydrochloric acid aqueous solution;
b) purification of the hydrochloride from the previous step by treatment with isopropyl alcohol;
c) crystallization of the hydrochloride by treatment with a hydrochloric acid aqueous solution.

## Patentansprüche

1. Verfahren zur Herstellung von 2-(3-Trifluormethylphenyl)-2-(2-benzoyloxyethylamino)propan unter Einschluß der Reaktion von m-Trifluormethylphenylaceton mit 2-Benzoylethanolaminhydrochlorid in Gegenwart von NaBH₄ und in DMF als Lösungsmittel.

2. Verfahren nach Anspruch 1 weiter umfassend
a) Behandlung des Rohprodukts (1), das aus dem Verfahren nach Anspruch 1 erhalten wurde, mit einer wässrigen Salzsäurelösung;
b) Aufreinigung des Hydrochlorids aus dem vorangehenden Schritt durch die Behandlung mit Isopropylalkohol;
c) Kristallisation des Hydrochlorids durch Behandlung mit einer wässrigen Salzsäurelösung.

## Revendications

1. Procédé de préparation du 1-(3-trifluorométhylphényl)-2-(2-benzoyloxyéthylamino)propane, comprenant la réaction entre de la m -trifluorométhylphénylacétone et du chlorhydrate de 2-benzoyléthanolamine en présence de NaBH₄ et dans du DMF en tant que solvant.

2. Procédé selon la revendication 1, comprenant en outre :
a) le traitement du composé brut (1) obtenu selon le procédé de la revendication 1 avec une solution aqueuse d'acide chlorhydrique ;
b) la purification du chlorhydrate provenant de l'étape précédente par traitement avec de l'alcool isopropylique ;
c) la cristallisation du chlorhydrate par traitement avec une solution aqueuse d'acide chlorhydrique.
